# EUROPEAN PATENT SPECIFICATION

(11) **EP 4 426 229 B1**
(45) Date of publication and mention of the grant of the patent: **09.07.2025**
(21) Application number: 22843879.2
(22) Date of filing: 19.12.2022
(51) Int. Cl.: A61C 1/08, A61B 34/20

(54) **ORAL SURGERY SYSTEM**
MUNDCHIRURGIESYSTEM
SYSTÈME DE CHIRURGIE BUCCALE

(30) Priority: 20.12.2021 IT 202100031862
(43) Date of publication of application: 11.09.2024
(73) Proprietor: Cavenago, Mauro, 35109 Montaña La Data Alta (Las Palmas) (ES); Panetta, Davide, 10137 Torino (TO) (IT); Baggi, Luigi, 00136 Roma (IT)
(72) Inventor: CAVENAGO, Mauro, 35109 Montaña la Data Alta (las Palmas de Gran Canaria) (ES); PANETTA, Davide, 10137 Torina (IT); BAGGI, Luigi, 00136 Roma (IT)
(74) Representative: Praxi Intellectual Property Milano
(86) International application number: PCT/IB2022/062465
(87) International publication number: WO 2023/119116

(56) References cited:
- US-A1- 2004 157 188
- US-A1- 2013 122 463
- US-A1- 2019 262 098
- US-A1- 2019 343 598

## Description

### FIELD OF THE ART

The present invention relates to an oral surgery system.

### STATE OF THE ART

Oral surgery, also known as odontostomatology, is the medical specialisation that deals with all those dental problems for which surgery is necessary, and also includes implantology and periodontology.

The oral surgeon deals, for example, with: dental extractions, removal of gum cysts, reconstruction of the jawbone, dental bone grafting, gum reconstruction, cosmetic dental procedures, dental implantology, and dental prostheses.

With reference to implantology, thanks to modern biomedical imaging techniques (such as T.A.C. Cone Beam), the oral surgeon is able to virtually assess and plan the insertion of dental, nasal, pterygoid or zygomatic implants, according to the most appropriate position and angle, depending on the patient's anatomical condition.

It should be noted that the angle of implant placement plays a fundamental role for the surgeon and prosthetist, as implant rehabilitation protocols and dental implant systems require/provide prosthetic components with predetermined angles (e.g., for totally edentulous jaws, protocols provide for implant placement at 0°, 17°, 30°, 45°, 60° depending on the type and number of implants placed).

Two main techniques for controlled implant placement are known: the technique with physical surgical guidance (e.g., that marketed by the company Materialise) and the technique based on an optical imaging system (e.g., that marketed by the company X-navtech).

The technique with a physical surgical guide involves making a template, usually of plastic material, with guide cylinders inside it that allow the osteotomy instruments to be used at a defined angle (estimated from a previous implant planning phase).

The Applicant notes that this technology implies significant and non-recoverable costs as each patient requires his own custom-made guide. Moreover, this technique is inaccurate in the case of totally edentulous jawbone due to the lack of anatomical references and the gingival support of the template itself. With regard to this imprecision, there have been reported cases of deaths due to an accidental perforation of the lingual artery caused by an imprecision in the insertion angle of the bur, which led to the perforation of the lingual wall of the mandible, resulting in hemorrhaging and death by suffocation of the patient. It should be noted that this technology involves rotating instruments to perform the osteotomy and does not allow the use of, for example, sonic and ultrasonic instruments. In addition, the surgical guide is located exactly above the surgical field, limiting the surgeon's view of it.

The technique based on the optical system allows, by means of passive optical sensors positioned on the surgical instrument and on the patient's anatomy, the position of the osteotomy instrument to be estimated in real time by means of image processing on the screen, creating a virtual correspondence (on the monitor) of the position of the instrument in relation to the Cone Beam T.A.C. It should be noted that the operator must constantly monitor the operating field and monitor and the cost of such equipment is high. Since the system is optical, any physical barriers between the passive optical sensors and the detector (active infrared sensors are usually used) affect the efficiency of detection.

In US2013/122463 there is provided a system and method that includes (i) transmitting a signal, (ii) receiving (a) a first reflection of the signal from a first reflector on a dental appliance, and (b) a second reflection of the signal from a second reflector on a dental tool, and (iii) determining, from the first reflection and the second reflection, a position of the second reflector relative to the first reflector, thus yielding a relative position of the second reflector.

### SUMMARY OF THE INVENTION

The technical problem addressed by the present invention is to propose an alternative oral surgery system to those known and which solves, at least in part, the problems mentioned above relating to the risks of inaccuracy, high cost and complexity.

The Applicant has perceived that the above-mentioned problem can be solved by employing a first sensor that provides data representative of the orientation of the surgical instrument and a second sensor that provides data representative of the orientation of the anatomical part involved; such orientation data make it possible to obtain information regarding the inclination of the surgical instrument with respect to the anatomical part of interest.

In particular, it is an object of the present invention to provide an oral surgery system as defined by claim 1 and particular embodiments thereof described by dependent claims 2-8.

### BRIEF DESCRIPTION OF THE FIGURES

The constructive and functional characteristics of the invention will be better understood from the detailed description below, in which reference is made to the enclosed drawing sheets representing certain preferred and non-limiting embodiments, wherein
- figure 1 schematically illustrates an oral surgery system comprising an instrumental sensor and an anatomical sensor;
- figure 2 shows an example of attachment of the anatomical sensor to a mandible of a totally edentulous patient, in particular, by means of osteosynthesis screws;
- figure 3 shows a partially edentulous mandibular arch equipped with an anatomical sensor and a surgical instrument equipped with an instrumental sensor;
- figure 4 refers to a way of using a calibration sensor;
- figure 5 shows an example of a representation of angular values made available by said oral surgery system.

### DETAILED DESCRIPTION

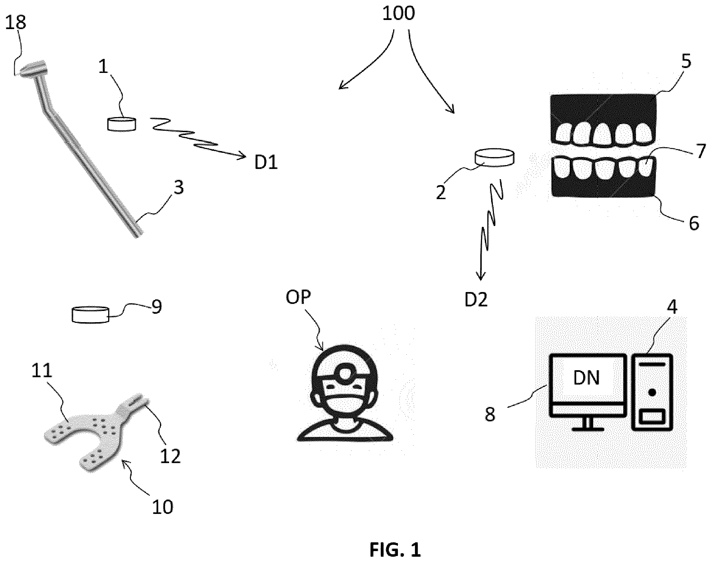

Figure 1 schematically shows a particular embodiment of an oral surgery system 100 comprising a first sensor 1, a second sensor 2, at least one surgical instrument 3 and a computing device 4.

The oral surgery system 100 allows the operator OP to perform a surgery on a patient of which the maxillary bone 5, the mandible 6 and some teeth 7 (which may be missing, in whole or in part) are schematically shown. For the purposes of the present invention, the term "oral surgery" refers to odontostomatological surgery including implantology and periodontology. For example, with reference to implantology, the oral surgery system 100 is employable to perform the insertion of dental implants. Surgical instrument 3 can be handled manually by the operator OP. The surgical instrument 3 is provided with a processing head 18. According to a particular embodiment shown in the figures, the surgical instrument 3 is an osteotomy instrument in which the processing head 18 is a rotary bur. According to other embodiments, the surgical instrument 3 is a nonrotating type such as, for example, a sonic, ultra-sonic or magnetically actuated instrument.

The first sensor 1 (referred to hereinafter as the "instrumental sensor") is attached to the surgical instrument 3 and is configured to measure the orientation in space of the surgical instrument itself and provide first data D1 representative of the measurements made. In particular, the instrumental sensor 1 is configured to measure the orientation in space of the rotary bur 18, for example, by indicating the angle or angles of inclination of a Z-axis of rotation of the rotary bur with respect to a reference. Alternatively, the instrumental sensor 1 may measure the orientation in space of the machining head 18 of the other types of surgical instruments used.

Such an instrumental sensor 1, in particular of a non-optical type, includes at least one of the following sensor devices: an accelerometer, a magnetometer, a combination of accelerometer and magnetometer (so-called electronic compass or e-compass), an inertial sensor, a gyroscope. In particular, instrumental sensor 1 is capable of providing a solid angle or the value of three angles that identify the orientation of a body in space (for example, the angles: pitch, yaw and roll) with respect to a reference.

The instrumental sensor 1 is equipped with a communication module capable of communicating with the computing device 4, for example, by means of a wireless communication mode and according to a short-range technology (for example, the Bluetooth standard). The instrumental sensor 1 is housed in an enclosure advantageously coated with a material that ensures impermeability to liquids and impurities. For example, the covering material is a plastic type such as, preferably, rubber or polymeric material. In particular, instrumental sensor 1 is equipped with a rechargeable battery and has adequately sealed electrical contact terminals.

The instrumental sensor 1 is fixed to the surgical instrument 3 by means of fixing means which may include adhesives, mechanical devices (for example, screws or interlocking shapes), magnetic elements. Preferably, such means of fixation are reversible and allow detachment of the instrumental sensor 1 from the surgical instrument 3. According to an exemplary implementation form, the instrumental sensor 1 has a cylindrical or disc shape with a diameter of about 15mm, and a thickness of about 5mm.

The second sensor 2 (hereinafter referred to as "anatomical sensor") is attachable to the dentition (i.e. to one or more teeth 7, if any) or to an area of the patient's maxillary bone 5 or jaw 6 and is configured to measure the orientation in space of the patient's maxillary bone 5 or jaw 6 with respect to a reference plane and provide second data D2 representative of the measurements made. Such an anatomical sensor 2 is structurally analogous or identical to the instrumental sensor 1 and capable of communicating with the computing device 4.

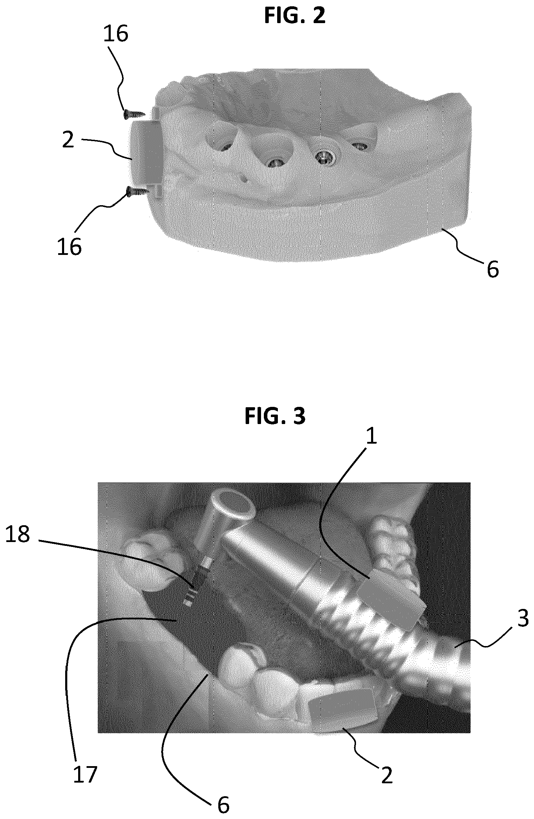

For example, the anatomical sensor 2 is fixable to one or more teeth 7 (if present) or to other areas of the maxillary bone 5 or of the mandible 6 by means of mechanical devices such as, for example, common dental dike hooks or hooks6 (for example, in the case of the presence of residual teeth of the patient) or screws (also known by the name of pins) 16 transgingival (in the case of totally edentulous maxillary or mandibular bones), as shown in the example in Figure 2. Note that the instrumental sensor 1 and the anatomical sensor 2 can be mounted in such a way that they do not obstruct the view of the areas of the patient's mouth to be worked on by the operator OP.

The computing device 4 receives the first data D1 and the second data D2 and, from them, calculates at least one angular value DN representative of an inclination between said surgical instrument 3 and the maxillary bone 5 or the mandible 6. The calculated data DN may also express the position of the surgical instrument 3 with respect to the maxillary bone 5 or the mandible 6.

The computing device 4 is realisable from a computer, such as a personal computer (laptop, desktop) or a management computer of a dental surgical room, in which appropriate calculation software resides. The computing device 4 is equipped with one or more user interfaces, such as a monitor 8 (e.g., in LED, LCD, OLED technology), which allow the operator OP to observe the point values of the inclination DN. It should be noted that, according to further embodiments, the calculation of the inclination DN or the display of the calculated value may be performed via a tablet or smartphone equipped with an appropriate application.

According to a particular example, the computing device 4 is such as to calculate one or more values relating to one or more angles between a first reference axis associated with the surgical instrument 3 and a second reference axis associated with the maxillary bone 5 or the mandible 6. In the case of skewed axes, several angles may be defined to identify their relative orientation or inclination. The first axis associated with the surgical instrument 3 is, for example, the axis of rotation of the bur 18 or, for other types of instruments, is an axis associated with the working head of that surgical instrument. The second axis is, for example, an axis orthogonal to the patient's occlusal board. As is well known, the occlusal table is made up of all the chewing surfaces of the upper or lower teeth, including the anterior teeth.

According to a particular form of realisation, the projection of the angle between the axes can be calculated according to a cross-section line and according to a panorex line. These two types of angle values are familiar to the technician as they are common to those used for the Cone Beam T.A.C. Monitor 8 allows the operator OP to read off the measured values of these angle types.

In accordance with an exemplary embodiment, the values calculated by the computing device 4 include the solid angle between the surgical instrument 3 and the anatomical part of interest and/or one or more projections of the solid angle according to particular projection planes (such as, for example, those of the cross section and/or panorex lines).

It should be noted that the instrumental sensor 1 and the anatomical sensor 2 are suitably calibrated so that the data measured by them are expressed in a common reference system and, preferably, are expressed not with absolute values but with relative values, which identify the inclination of the surgical instrument with respect to the anatomical area of interest.

With regard to calibration and according to a preferred form of implementation, the oral surgery system 100 may further be equipped with a third sensor 9 (hereinafter referred to as, calibration sensor), which is structurally identical or analogous to the instrumental sensor 1 and the anatomical sensor 2 and configured to carry out a calibration procedure of the instrumental sensor 1 and the anatomical sensor 2. The calibration sensor 9 is in communication (for example, by means of Bluetooth technology) with the instrumental sensor 1 and the anatomical sensor 2, as well as with the computing device 4.

The calibration procedure carried out using the calibration sensor 9 allows the data measured by the instrumental sensor 1 and the anatomical sensor 2 to be expressed with respect to a common reference plane, such as the patient's occlusal plate.

For example, this procedure, requires the calibration sensor 9 to be physically associated with the maxillary bone 5 and the mandible 6, in order to detect the patient's occlusal plateau that plays the role of reference plane. In this case, the calibration sensor 9 is mounted on a support device 10 (of disposable or sterilisable type) which is temporarily placed in contact with the residual teeth 7 or with the gingiva associated with the maxillary bone 5 and/or the mandible 6.

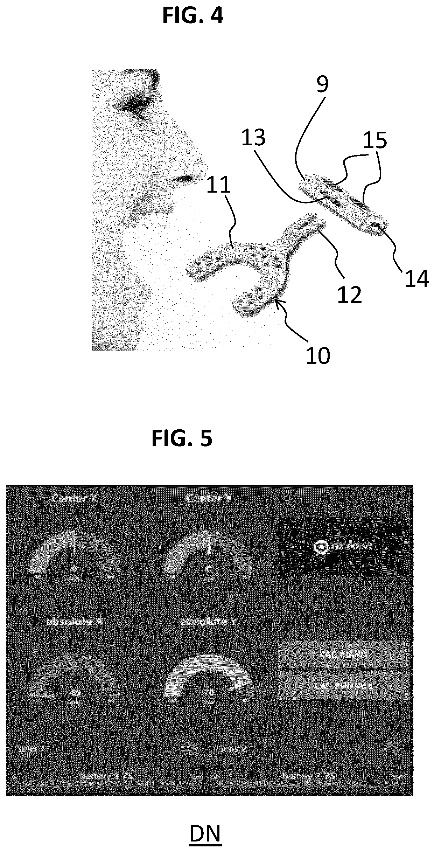

According to a particular form of implementation (see Figure 5), such a support device 10 may comprise a bite registration rim (occlusal plate) 11 integral with a dental fork 12. The calibration sensor 9, the casing of which may be provided with an opening 13, is mounted to the dental fork 12 via said opening 13. The bite registration rim 11 is, advantageously, a negative of the patient's occlusal board so as to ensure a correct fit on the residual dentition or gingival mucosa.

In the calibration, the bite registration rim 11 is then clamped with the teeth 7 (if present) between the patient's maxilla 5 and mandible 6, thus allowing the calibration sensor 9 to record the reference values defining the occlusal board.

Furthermore, the calibration sensor 9 communicates (directly or via the computing device 4) with the anatomical sensor 2 so that the detected reference plane also results as the reference plane for the anatomical sensor 2 (alignment procedure).

In addition, the calibration procedure involves the detection by the calibration sensor 9 of an initial orientation (the zero point) assumed by the instrumental sensor 1 and its correlation to the occlusal plate. For this detection, the surgical instrument 3 is placed in a predetermined position that is already known to the calibration sensor 9 and/or the computing device 4. For example, in order to perform the "zero point" detection, the surgical instrument 3 is placed in a guide bushing of the support device 10 into which the bur 18 is inserted, or a shaped bed on which the bur 18 is placed, laterally.

According to one example, the calibration sensor 9 can also perform the function of an electrical charging device for the other two sensors 1 and 2. According to this example (figure 5), the calibration sensor 9 is provided with a charging port 14 for its own battery (e.g., USB-C type) and pins 15 for charging the instrumental sensor 1 and the anatomical sensor 2. According to an example embodiment, the calibration sensor 9 has dimensions of 20 mm x 40 mm x 10 mm.

It should be noted that the sensors described (instrumental sensor 1, anatomical sensor 2 and calibration sensor 9) thanks to the use of the coating described above are sterilisable and therefore reusable for other surgical procedures, even for different patients.

As regards the operation of the system 100, after having carried out the calibration procedure and having mounted the instrumental sensor 1 and the anatomical sensor 2 as described above, the operator OP can proceed to use the instrument 3 according to the type of intervention required.

It should be noted that, for example, the operator OP knows the values of the correct angles for the implants to be inserted, as implant rehabilitation protocols and implant dentistry systems require/provide prosthetic components with predetermined angles (e.g., for totally edentulous jaws the protocols provide for implant placement at 0°, 17°, 30°, 45°, 60° depending on the type and number of implants placed).

Figure 3 refers to an implantology operation and shows, schematically, a mandible 6 having an edentulous zone 17 and a portion of the dentition to which the anatomical sensor 2 is fixed (e.g. by means of a dental dam hook). On the edentulous zone 17 acts the surgical instrument 3 on which the instrumental sensor 1 is mounted.

At predetermined intervals, the instrumental sensor 1 and the anatomical sensor 2 transmit the measured data D1 and D2 (relating to the absolute orientation of the surgical instrument 3 and/or the mandible 6) to the computing device 4, which calculates the data relating to the inclination of the surgical instrument 3 with respect to the mandible 6 according to a desired type. The data relating to this inclination changes with each movement of the surgical instrument 3 and each movement of the mandible 6. The angular values identifying this inclination are calculated in real time and shown to the operator OP via the monitor 8.

Figure 5 shows, by way of example, how monitor 8, associated with computing device 4, can make the angular information of interest visible to the operator OP during surgery. The operator OP (according to the example, an implantologist) is able to assess the positions and angles of implant insertion in consideration of the anatomical conditions of the patient and thus, observing the values indicated on the monitor 8, manoeuvres the surgical instrument 3 in the most correct way.

It should be noted that, advantageously, by considering as the surgical instrument 3 the bur 18 rotating around the Z axis, the calculation method employed by the computing device 4 can be such as to cancel, from the measurement made by the instrumental sensor 2, the angular variations due to a rotation of the instrument 3 around said Z axis made by the operator OP. The rotation of the surgical instrument 3 around the Z-axis may be necessary to adapt to the different anatomical areas of the concerned zone, but such a rotation must not affect the measured angle (inclination). It is also possible not to provide this option, for example, in relation to sonic, ultrasonic or magnetically actuated shaped instruments.

The oral surgery system 100 can also be used for extra-oral and, in particular, head-related surgeries performed with instruments similar to those used in dental environments, e.g. to position implants that act as anchorage points for nasal prostheses, the auricle, the eye socket, the zygomatic massif, etc., for patients undergoing anatomical resections, due to congenital deformities or following traumatic events. In this case, the anatomical sensor 2 is made integral with the bone area of interest.

The oral surgery system 100 described above is particularly advantageous in that it is a valuable support for the surgeon's work and, at the same time, is neither particularly complex nor particularly expensive.

The low cost of the system 100 is attributable to the low cost of the sensors used and also the possibility of reusing them.

In addition, the system 100 described above allows the surgeon to operate with due precision as he is constantly informed about the inclinations of the surgical instruments handled, without the need to install guides and even in edentulous areas (and therefore in the absence of natural references), avoiding the serious problems related to accidental perforations.

Compared to the technique based on the use of optical sensors, the system 100 described above is less complex and less expensive as the calculation software does not require image processing or integration with images provided by other diagnostic instruments, e.g. T.A.C. Cone Beam.

It should also be noted that the positioning of the instrumental sensor 1 and the anatomical sensor 2 provided by the system 100 can be carried out in a way that does not restrict the surgeon's view.

### LEGEND OF FIGURE REFERENCE NUMBERS

- oral surgery system 100
- first sensor (instrumental sensor) 1
- second sensor (anatomical sensor) 2
- surgical instrument 3
- operator OP
- computing device 4
- maxillary bone 5
- mandible 6
- teeth 7
- first data D1
- second data D2
- monitor 8
- inclination DN
- calibration sensor 9
- support device 10
- bite registration rim 11
- dental fork 12
- opening 13
- charging port 14
- pin 15
- screws 16
- edentulous zone 17
- rotary bur 18

## Claims

1. Oral surgery system (100) comprising:
a surgical instrument (3) movable by an operator (OP);
a first sensor (1) attached to the surgical instrument (3) and configured to provide first data (D1) representative of an orientation in space of the surgical instrument (3);
a second sensor (2) so as to be attached to the dentition (7) or maxillary bone (5) or mandible (6) of the patient and provide second data representative of an orientation in space of the maxillary bone (5) or mandible (6) of the patient;
a computing device (4) connected to the first (1) and second (2) sensors to receive the first (D1) and second (D2) data and configured to provide at least one angular value (DN) representative of an inclination between said surgical instrument (3) and the maxillary bone (5) or mandible (6);
an interface device (8) configured to make available to the operator (OP) said at least one angular value representative of the inclination;
**characterized by** further comprising:
a third sensor (9) configured to communicate with the first sensor (1) and the second sensor (2) and perform a calibration procedure comprising:
detecting an orientation of a plane defined by the maxillary bone (5) and/or the mandible (6) and associating it as a reference plane for the second sensor (2);
detecting an initial value associated with an orientation of the first sensor (1) attached to the surgical instrument (3) and correlating it to that reference plane.

2. System (100) according to claim 1, wherein:
the first (1) and the second (2) sensors respectively include at least one of the sensors belonging to the group: an accelerometer, a magnetometer, a combination of accelerometer and magnetometer, an inertial sensor.

3. System (100) according to claim 1, wherein:
- said at least one angular value (DN) includes at least one of: one or more values relating to one or more angles between a first reference axis (Z) associated with the surgical instrument (3) and a second reference axis associated with the occlusal plane of the patient;
or
- wherein said at least one angular value (DN) comprises at least one of: a projection of an angle according to a reference plane; a projection of an angle according to a cross-sectional line; a projection of an angle according to a panorex line;
or
- a solid angle, a projection of a solid angle according to a reference plane; a projection of a solid angle according to a cross-section line, a projection of a solid angle according to a panorex line.

4. System (100) according to at least one of claims 1, wherein at least one of said sensors (1, 2, 9) comprises:
a container coated with a material that ensures impermeability from liquids or impurities.

5. System (100) according to claim 1, further comprising:
a disposable or sterilizable support device (10) of said third sensor (9) such that it is physically associated with the maxillary bone (5) and/or the mandible (6) to perform said calibration procedure.

6. System (100) according to claim 5, wherein the support device comprises (10):
an occlusal plate (11) configured to be clamped between the maxillary bone (5) and the mandible (6) of the patient;
a connecting element (12) connected to said occlusal plate (11) and configured to be connected to the third sensor (9).

7. System (100) according to claim 1wherein:
at least one of said sensors (1, 2, 9) is configured to communicate with said computing device (4) in a wireless mode.

8. System (100) according to claim 1, further comprising at least one device for attaching said second sensor (2) to the maxillary bone (5), or to at least one tooth or jaw (6) of the patient.

## Patentansprüche

1. Oralchirurgisches System (100), umfassend:
ein chirurgisches Instrument (3), das durch einen Bediener (OP) bewegbar ist;
einen ersten Sensor (1), der an dem chirurgischen Instrument (3) angebracht ist und konfiguriert ist, um erste Daten (D1), die eine Ausrichtung des chirurgischen Instruments (3) in einem Raum darstellen, bereitzustellen;
einen zweiten Sensor (2), der an dem Gebiss (7) oder dem Oberkieferknochen (5) oder dem Unterkiefer (6) des Patienten angebracht wird und zweite Daten, die eine Ausrichtung des Oberkieferknochens (5) oder Unterkiefers (6) des Patienten in dem Raum darstellen, bereitstellt;
eine Rechenvorrichtung (4), die mit dem ersten (1) und dem zweiten (2) Sensor verbunden ist, um die ersten (D1) und die zweiten (D2) Daten zu empfangen, und die konfiguriert ist, um mindestens einen Winkelwert (DN), der eine Neigung zwischen dem chirurgischen Instrument (3) und dem Oberkieferknochen (5) oder dem Unterkiefer (6) darstellt, bereitzustellen;
eine Schnittstellenvorrichtung (8), die konfiguriert ist, um dem Bediener (OP) den mindestens einen Winkelwert, der die Neigung darstellt, zur Verfügung zu stellen;
**gekennzeichnet durch** ferner umfassend:
einen dritten Sensor (9), der konfiguriert ist, um mit dem ersten Sensor (1) und dem zweiten Sensor (2) zu kommunizieren und ein Kalibrierungsverfahren durchzuführen, umfassend:
Erfassen einer Ausrichtung einer Ebene, die durch den Oberkieferknochen (5) und/oder den Unterkiefer (6) definiert ist, und Verknüpfen derselben als eine Bezugsebene für den zweiten Sensor (2);
Erfassen eines Anfangswerts, der mit einer Ausrichtung des ersten Sensors (1), der an dem chirurgischen Instrument (3) angebracht ist, verknüpft ist, und Korrelieren desselben mit dieser Bezugsebene.

2. System (100) nach Anspruch 1, wobei:
der erste (1) beziehungsweise der zweite (2) Sensor mindestens einen von den Sensoren, die zu der Gruppe gehören: einen Beschleunigungsmesser, ein Magnetometer, eine Kombination aus Beschleunigungsmesser und Magnetometer, einen Trägheitssensor, einschließen.

3. System (100) nach Anspruch 1, wobei:
- der mindestens eine Winkelwert (DN) mindestens eines einschließt von: einem oder mehreren Werten, die sich auf einen oder mehrere Winkel zwischen einer ersten Bezugsachse (Z), die mit dem chirurgischen Instrument (3) verknüpft ist, und einer zweiten Bezugsachse, die mit der okklusalen Ebene des Patienten verknüpft ist, beziehen;
oder
- wobei der mindestens ein Winkelwert (DN) mindestens eines umfasst von: einer Projektion eines Winkels gemäß einer Bezugsebene; einer Projektion eines Winkels gemäß einer Querschnittslinie; einer Projektion eines Winkels gemäß einer Panorexlinie;
oder
- einem Raumwinkel, einer Projektion eines Raumwinkels gemäß einer Bezugsebene; einer Projektion eines Raumwinkels gemäß einer Querschnittslinie, einer Projektion eines Raumwinkels gemäß einer Panorexlinie.

4. System (100) nach mindestens einem von Anspruch 1, wobei mindestens einer von den Sensoren (1, 2, 9) umfasst:
einen Behälter, der mit einem Material, das Undurchlässigkeit gegenüber Flüssigkeiten oder Verunreinigungen gewährleistet, beschichtet ist.

5. System (100) nach Anspruch 1, ferner umfassend:
eine Einweg- oder sterilisierbare Trägervorrichtung (10) des dritten Sensors (9), derart, dass er mit dem Oberkieferknochen (5) und/oder dem Unterkiefer (6) physisch verknüpft ist, um das Kalibrierungsverfahren durchzuführen.

6. System (100) nach Anspruch 5, wobei die Trägervorrichtung umfasst (10):
eine okklusale Platte (11), die konfiguriert ist, um zwischen dem Oberkieferknochen (5) und dem Unterkiefer (6) des Patienten eingeklemmt zu werden;
ein Verbindungselement (12), das mit der okklusalen Platte (11) verbunden ist und konfiguriert ist, um mit dem dritten Sensor (9) verbunden zu werden.

7. System (100) nach Anspruch 1, wobei:
mindestens einer von den Sensoren (1, 2, 9) konfiguriert ist, um in einem Drahtlosmodus mit der Rechenvorrichtung (4) zu kommunizieren.

8. System (100) nach Anspruch 1, ferner umfassend mindestens eine Vorrichtung zum Anbringen des zweiten Sensors (2) an dem Oberkieferknochen (5) oder an mindestens einem Zahn oder Kiefer (6) des Patienten.

## Revendications

1. Système de chirurgie buccale (100) comprenant :
un instrument chirurgical (3) déplaçable par un opérateur (OP) ;
un premier capteur (1) fixé à l'instrument chirurgical (3) et configuré pour fournir des premières données (D1) représentant une orientation dans l'espace de l'instrument chirurgical (3) ;
un deuxième capteur (2) devant être fixé à la dentition (7) ou à l'os maxillaire (5) ou à la mandibule (6) du patient et fournir des secondes données représentant une orientation dans l'espace de l'os maxillaire (5) ou de la mandibule (6) du patient ;
un dispositif informatique (4) connecté aux premier (1) et deuxième (2) capteurs pour recevoir les premières (D1) et secondes (D2) données et configuré pour fournir au moins une valeur angulaire (DN) représentant une inclinaison entre ledit instrument chirurgical (3) et l'os maxillaire (5) ou la mandibule (6) ;
un dispositif d'interface (8) configuré pour mettre à la disposition de l'opérateur (OP) ladite au moins une valeur angulaire représentant l'inclinaison ;
**caractérisé en ce qu'**il comprend en outre :
un troisième capteur (9) configuré pour communiquer avec le premier capteur (1) et le deuxième capteur (2) et réaliser une procédure d'étalonnage comprenant :
la détection d'une orientation d'un plan défini par l'os maxillaire (5) et/ou la mandibule (6) et l'association dudit plan comme plan de référence pour le deuxième capteur (2) ;
la détection d'une valeur initiale associée à une orientation du premier capteur (1) fixé à l'instrument chirurgical (3) et la corrélation de ladite valeur initiale audit plan de référence.

2. Système (100) selon la revendication 1, dans lequel :
le premier (1) et le deuxième (2) capteur comportent respectivement au moins un des capteurs appartenant au groupe comprenant : un accéléromètre, un magnétomètre, une combinaison d'un accéléromètre et d'un magnétomètre, un capteur inertiel.

3. Système (100) selon la revendication 1, dans lequel :
- ladite au moins une valeur angulaire (DN) comporte au moins l'une des valeurs suivantes : une ou plusieurs valeurs se rapportant à un ou plusieurs angles entre un premier axe de référence (Z) associé à l'instrument chirurgical (3) et un second axe de référence associé au plan occlusal du patient ;
ou
- dans lequel ladite au moins une valeur angulaire (DN) comprend au moins l'un des éléments suivants : une projection d'un angle selon un plan de référence ; une projection d'un angle selon une ligne de coupe transversale ; une projection d'un angle selon une ligne de radiographie panoramique ;
ou
- un angle solide, une projection d'un angle solide selon un plan de référence ; une projection d'un angle solide selon une ligne de coupe transversale, une projection d'un angle solide selon une ligne de radiographie panoramique.

4. Système (100) selon au moins l'une des revendications 1, dans lequel au moins l'un desdits capteurs (1, 2, 9) comprend :
un récipient recouvert d'un matériau qui assure l'imperméabilité aux liquides ou aux impuretés.

5. Système (100) selon la revendication 1, comprenant en outre :
un dispositif de support (10) jetable ou stérilisable dudit troisième capteur (9) de sorte qu'il est physiquement associé à l'os maxillaire (5) et/ou à la mandibule (6) pour réaliser ladite procédure d'étalonnage.

6. Système (100) selon la revendication 5, dans lequel le dispositif de support (10) comprend :
une plaque occlusale (11) conçue pour être serrée entre l'os maxillaire (5) et la mandibule (6) du patient ;
un élément de liaison (12) relié à ladite plaque occlusale (11) et conçu pour être relié au troisième capteur (9).

7. Système (100) selon la revendication 1 dans lequel :
au moins l'un desdits capteurs (1, 2, 9) est configuré pour communiquer avec ledit dispositif informatique (4) dans un mode sans fil.

8. Système (100) selon la revendication 1, comprenant en outre au moins un dispositif pour la fixation dudit deuxième capteur (2) à l'os maxillaire (5), ou à au moins une dent ou une mâchoire (6) du patient.
